# EUROPEAN PATENT APPLICATION

(11) **EP 4 470 493 A1**
(43) Date of publication of application: **04.12.2024**
(21) Application number: 23743365.1
(22) Date of filing: 23.01.2023
(51) Int. Cl.: A61B 18/18

(54) **MEDICAL TREATMENT TOOL**

(30) Priority: 24.01.2022 JP 2022008340
(71) Applicant: Saney Seiko Inc., Asaka-shi, Saitama 351-0021 (JP); Nikkiso Co., Ltd., Tokyo 150-6022 (JP)
(72) Inventor: MANPUKU Yasuhiro, Asaka-shi, Saitama 351-0021 (JP); ISHIKAWA Hideki, Asaka-shi, Saitama 351-0021 (JP); ISHIZEKI Yasunori, Asaka-shi, Saitama 351-0021 (JP); MORISHITA Masahiro, Asaka-shi, Saitama 351-0021 (JP); OGIWARA Mariho, Asaka-shi, Saitama 351-0021 (JP); MATSUZAKI Masahiro, Asaka-shi, Saitama 351-0021 (JP); KUMAGAI Tomoki, Asaka-shi, Saitama 351-0021 (JP); KINOSHITA Yoshihiko, Tokyo 150-6022 (JP); ASANO Takuji, Tokyo 150-6022 (JP); NISHISE Naoya, Tokyo 150-6022 (JP)
(74) Representative: Schmidt, Christian
(86) International application number: PCT/JP2023/001842
(87) International publication number: WO 2023/140373

(57) **Abstract**

The related-art medical treatment instrument that allows radiation of a microwave from both electrodes has had one or more of the following various problems caused by the microwave radiation from the both electrodes. The various problems include, for example, (1) occurrence of scissoring (occurrence of sparks), (2) heat generation and radiation to a back side of a radiation surface in contact with the both electrodes, (3) inefficiency of the microwave radiation through use of a microwave splitter, (4) falling off of both antennas, (5) a weak gripping force, (6) difficulty in grabbing a thin tissue such as a membrane, and (7) a microwave radiation range being not able to be grasped easily. It has been confirmed that a configuration of a medical treatment instrument according to the present invention solves the one or more of the problems described above, and the present invention has been completed.

## Description

### Technical Field

The present invention relates to a medical treatment instrument which is capable of radiating a microwave from both electrodes having a scissoring prevention mechanism.

The present application claims priority from Japanese Patent Application No. 2022-008340, the disclosure of which is incorporated herein by reference.

### Background Art

### (Device Using Microwave)

There has been known that a microwave can coagulate (immobilize) biological tissue such as digestive organs, a liver, a bladder, a prostate, a uterus, blood vessels, or intestinal tracts at low temperature. Then, various devices for assisting surgery through use of the microwave have been developed.

### (Related Art)

As the devices using a microwave, the following plurality of devices have been reported.

In Patent Literature 1, there is disclosed "a medical treatment tool including: a first electrode including one or a plurality of microwave application antennas 1 and one or a plurality of microwave receiver antennas 1; a second electrode including one or a plurality of microwave application antennas 2 and one or a plurality of microwave receiver antennas 2; a first coaxial cable including a first central conductor and a first external conductor, the first central conductor and the first external conductor being connected directly or indirectly to the microwave application antenna 1 and the microwave receiver antenna 1, respectively; a second coaxial cable including a second central conductor and a second external conductor, the second central conductor and the second external conductor being connected directly or indirectly to the microwave application antenna 2 and the microwave receiver antenna 2, respectively; a microwave transmission coaxial cable; and a microwave splitter for splitting a microwave transmitted by the microwave transmission coaxial cable to the first central conductor and the second central conductor, in which the medical treatment tool can irradiate a microwave from both the first electrode and the second electrode."

In this Patent Literature, a microwave distribution circuit is disclosed, but a configuration of the medical treatment tool according to the present invention is not disclosed.

In Patent Literature 2, there is disclosed "a microwave surgical instrument including: a surgical instrument main body including an electrode portion for irradiating biological tissue with a microwave; a microwave oscillator which is provided in the surgical instrument main body, and is configured to oscillate a microwave; and an amplifier which is provided in the surgical instrument main body, and is connected between the electrode portion and the microwave oscillator, the amplifier being configured to amplify the microwave from the microwave oscillator and send the amplified microwave to the electrode portion, the microwave surgical instrument further including: a variable output matching circuit which is connected between the amplifier and the electrode portion, and is configured to match an output impedance of the amplifier and an impedance of the biological tissue with each other; a detection circuit configured to separately detect reflected power and incident power given between the amplifier and the electrode portion; and control means for controlling the variable output matching circuit based on values of the incident power and the reflected power detected by the detection circuit."

In this Patent Literature, the variable output matching circuit for matching the output impedance of the amplifier and the impedance of the biological tissue with each other is disclosed, but the feature that the microwave can be radiated from both electrodes of the medical treatment instrument according to the present invention and the configuration of the electrodes are not disclosed.

In Patent Literature 3, there is disclosed "electrosurgical forceps including: a pair of jaw elements pivotable relative to each other to open and close a gap therebetween; a first transmission line structure mounted in one of the pair of jaw elements adjacent to the gap; a second transmission line structure mounted in the other one of the pair of jaw elements adjacent to the gap opposite the first transmission line structure; a coaxial cable for conveying microwave frequency energy; and a power splitter at a distal end of the coaxial cable, the power splitter being arranged to divide the microwave frequency energy conveyed by the coaxial cable between the first transmission line structure and the second transmission line structure, in which each of the first transmission line structure and the second transmission line structure consists of an unbalanced lossy transmission line to support the microwave energy as a travelling wave, and in which each of the first transmission line structure and the second transmission line structure has an electrical length along the travelling wave that is non-resonant for the microwave energy."

In this Patent Literature, the feature that the microwave can be radiated from both electrodes is disclosed, but the configuration of the electrodes of the medical treatment instrument according to the present invention is not disclosed.

Consequently, none of the patent literatures discloses or suggests the electrode structure of the present invention that allows efficient radiation of a microwave from both electrodes.

### Citation List

### Patent Literature

[PTL 1] JP 2018/147243 A
[PTL 2] JP 2012-115384 A
[PTL 3] JP 2016-533862 A1

### Summary of Invention

### Technical Problem

The related-art medical treatment instrument that allows radiation of a microwave from both electrodes has had one or more of the following various problems caused by the microwave radiation from the both electrodes. The various problems include, for example, (1) occurrence of scissoring (occurrence of sparks), (2) heat generation and radiation to a back side of a radiation surface in contact with the both electrodes, (3) inefficiency of the microwave radiation through use of a microwave splitter, (4) falling off of both antennas, (5) a weak gripping force, (6) difficulty in grabbing a thin tissue such as a membrane, and (7) a microwave radiation range being not able to be grasped easily.

### Solution to Problem

As a result of extensive studies conducted to solve one or more of the problems described above, the inventors of the present invention have confirmed that the configuration of the medical treatment instrument according to the present invention solves the one or more of the problems described above, and has completed the present invention.

That is, the present invention includes the following.
1. A medical treatment instrument including:
   a first electrode including a microwave application antenna A and a microwave reception antenna B;
   a second electrode including a microwave application antenna C and a microwave reception antenna D;
   a first coaxial cable including a first central conductor and a first external conductor, a distal end of the first central conductor and a distal end of the first external conductor being directly or indirectly connected to a terminal end of the microwave application antenna A and a terminal end of the microwave reception antenna B, respectively;
   a second coaxial cable including a second central conductor and a second external conductor, a distal end of the second central conductor and a distal end of the second external conductor being directly or indirectly connected to a terminal end of the microwave application antenna C and a terminal end of the microwave reception antenna D, respectively;
   a microwave transmission coaxial cable including a central conductor and an external conductor;
   a microwave splitter including a conductor for splitting having a branch 1 and a branch 2, the branch 1 and the branch 2 being formed into a two-legged fork shape on a distal end side of the microwave splitter, a terminal end of the conductor for splitting being directly or indirectly connected to the central conductor of the microwave transmission coaxial cable, the branch 1 being directly or indirectly connected to a terminal end of the first central conductor, and the branch 2 being directly or indirectly connected to a terminal end of the second central conductor; and
   position setting portions of a first electrode and a second electrode, the position setting portions including a position setting portion A and a position setting portion B being opposed to each other, the position setting portion A being arranged in the first electrode including a fitting protruding portion A, a fitting hole portion A, and an accommodation portion A that accommodates the first coaxial cable, and a position setting portion B being arranged in the second electrode including a fitting protruding portion B, a fitting hole portion B, and an accommodation portion B that accommodates the second coaxial cable, the fitting protruding portion A and the fitting protruding portion B being configured to be accommodated in the fitting hole portion B and the fitting hole portion A, respectively, when the first electrode and the second electrode are brought into contact with each other.
2. The medical treatment instrument according to the above-mentioned item 1, further including an antenna cover A,
   wherein the antenna cover A includes a distal-end side inner cavity fitting hole portion in an inner cavity on a distal end side of the cover,
   wherein the microwave application antenna A and/or the microwave application antenna C includes a distal-end side fitting protruding portion at an upper end on a distal end side of the antenna, and
   wherein the distal-end side fitting protruding portion is fitted into the distal-end side inner cavity fitting hole portion.
3. The medical treatment instrument according to the above-mentioned item 1 or 2,
   wherein the antenna cover A includes a distal-end side outer engagement portion on a distal end side of the cover,
   wherein the microwave reception antenna B and/or the microwave reception antenna D includes a distal-end side receiving portion of the antenna, and
   wherein the distal-end side outer engagement portion is engaged with the distal-end side receiving portion.
4. The medical treatment instrument according to the above-mentioned item 2 or 3, further including an antenna cover B,
   wherein the antenna cover B is located in an inner cavity of the microwave reception antenna B and/or the microwave reception antenna D, and accommodates a terminal end of the microwave application antenna A and/or the microwave application antenna C in an inner cavity of the cover.
5. The medical treatment instrument according to the above-mentioned item 4,
   wherein the antenna cover B has a key shape, and
   wherein the inner cavity of the microwave reception antenna B and/or the microwave reception antenna D has a keyhole shape corresponding to the key shape.
6. The medical treatment instrument according to the above-mentioned item 4 or 5,
   wherein a distal end of the antenna cover B includes an engagement portion 1,
   wherein an inner cavity of the antenna cover A includes an engagement portion 2, and
   wherein the engagement portion 1 and the engagement portion 2 are engaged with each other.
7. The medical treatment instrument according to any one of the above-mentioned items 1 to 6,
   wherein the terminal end of the conductor for splitting includes a fitting hole portion,
   wherein a distal end of the central conductor includes a fitting protruding portion, and
   wherein the fitting protruding portion is fitted into the fitting hole portion.
8. The medical treatment instrument according to any one of the above-mentioned items 1 to 7,
   wherein a distal end of the branch 1 of the conductor for splitting includes a fitting hole portion, the terminal end of the first central conductor includes a fitting protruding portion, and the fitting protruding portion is fitted into the fitting hole portion, and
   wherein a distal end of the branch 2 of conductor for splitting includes a fitting hole portion, the terminal end of the second central conductor includes a fitting protruding portion, and the fitting protruding portion is fitted into the fitting hole portion.
9. The medical treatment instrument according to any one of the above-mentioned items 1 to 8, wherein an internal angle formed by the branch 1 and the branch 2 is from 20° to 80°.
10. The medical treatment instrument according to any one of the above-mentioned items 1 to 9, wherein a surface of the microwave application antenna A and a surface of the microwave application antenna C each have a sawtooth shape, and are configured such that, when the first electrode and the second electrode are brought into contact with each other, the surfaces contact with each other only with a blade of the sawtooth shape of the microwave application antenna A and a blade of the sawtooth shape of the microwave application antenna C.
11. The medical treatment instrument according to any one of the above-mentioned items 1 to 10, wherein a distal end surface of the microwave reception antenna B and a distal end surface of the microwave reception antenna D each have a tooth shape, and are configured such that, when the first electrode and the second electrode are brought into contact with each other, the distal end surfaces mesh with each other only with the tooth shape of the distal end of the microwave reception antenna B and the tooth shape of the distal end of the microwave reception antenna D.
12. The medical treatment instrument according to any one of the above-mentioned items 1 to 11,
   wherein the antenna cover A includes a marking at a specific position from a distal end of the first electrode or the second electrode, on a surface of the cover A.
13. The medical treatment instrument according to any one of the above-mentioned items 1 to 12,
   wherein the microwave splitter further includes:
      a casing including two casing covers, the casing covers each having an outer cover arranged therein;
      two insulator (dielectric) covers;
      two metal covers; and
      two resin covers,
   wherein the two resin covers are arranged so as to sandwich the conductor for splitting therebetween,
   wherein the two metal covers are arranged so as to sandwich the two resin covers therebetween,
   wherein the two insulator (dielectric) covers are arranged so as to sandwich the two metal covers therebetween, and
   wherein the two casing covers are configured so as to sandwich the two insulator (dielectric) covers therebetween.

### Advantageous Effects of Invention

The medical treatment instrument according to the present invention has one or more of the following effects.
(1) suppressing occurrence of scissoring (occurrence of sparks)
(2) suppressing heat generation and radiation to a back side of a radiation surface in contact with the both electrodes
(3) eliminating inefficiency of the microwave radiation through use of a microwave splitter
(4) preventing falling off of both antennas
(5) a strong gripping force
(6) ease in grabbing a thin tissue such as a membrane
(7) a microwave radiation range being able to be grasped easily

### Brief Description of Drawings

FIG. 1A is an overall side view of a medical treatment instrument (1). FIG. 1B is a sectional view taken along the line C-C of the FIG. 1A.
FIG. 2A is a view of a first electrode (4) when seen from the arrow A (enlarged view) of the medical treatment instrument (1). FIG. 2B is a view of a second electrode (7) when seen from the arrow B (enlarged view) of the medical treatment instrument (1) .
FIG. 3A is an overall side view of the medical treatment instrument (1). FIG. 3B is a sectional view taken along the line D-D of the FIG. 3A.
FIG. 4A is an overall view of the first electrode (4) and the second electrode (7). FIG. 4B is a view of the second electrode (7) when seen from the arrows E.
FIG. 5 is a schematic view of a microwave application antenna A (2), a microwave application antenna C (5), a microwave reception antenna B (3), a microwave reception antenna D (6), an antenna cover A (30), and an antenna cover B (38).
FIG. 6A is an explanatory view for illustrating a state in which the microwave application antenna A (2) or the microwave application antenna C (5) is to be inserted into the antenna cover A (30) in the arrow direction. FIG. 6B is a sectional view of the antenna cover A (30) into which the microwave application antenna A (2) or the microwave application antenna C (5) has been inserted.
FIG. 7A is an explanatory view for illustrating a state in which the antenna cover A (30) is to be inserted into the microwave reception antenna B (3) or the microwave reception antenna D (6) in the arrow direction. FIG. 7B is a sectional view of the microwave reception antenna B (3) or the microwave reception antenna D (6) into which the antenna cover A (30) has been inserted.
FIG. 8A is an explanatory view for illustrating a state in which the antenna cover B (38) is to be inserted into an inner cavity of the microwave reception antenna B (3) or the microwave reception antenna D (6). FIG. 8B is a sectional view of the microwave reception antenna B (3) or the microwave reception antenna D (6) into which the antenna cover B (38) has been inserted.
FIG. 9 is an explanatory view of the antenna cover B (38) of a key shape (40) having a round shape, and the microwave reception antenna B (3) and/or the microwave reception antenna D (6) that includes an inner cavity having a keyhole shape (39).
FIG. 10 is an explanatory view for illustrating a state in which the antenna cover B (38) is located in the inner cavity of the microwave reception antenna B (3) and/or the microwave reception antenna D (6).
FIG. 11 is an explanatory view of an engagement portion 1 (55) and an engagement portion 2 (56).
FIG. 12 is an explanatory view for illustrating a region in which the engagement portion 1 (55) and the engagement portion 2 (56) are engaged with each other (engagement portion (54)).
FIG. 13 is an explanatory view of a publicly known microwave splitter (17).
FIG. 14 is an explanatory view of the microwave splitter (17) having a preferred internal angle (51).
FIG. 15 is an explanatory view of microwave application antennas each having a sawtooth shape and microwave reception antennas that mesh with each other only with tooth shapes at distal ends thereof.
FIG. 16 is an explanatory view of a marking (63).
FIG. 17A and 17B are a configuration example of the microwave splitter (17).

### Description of Embodiments

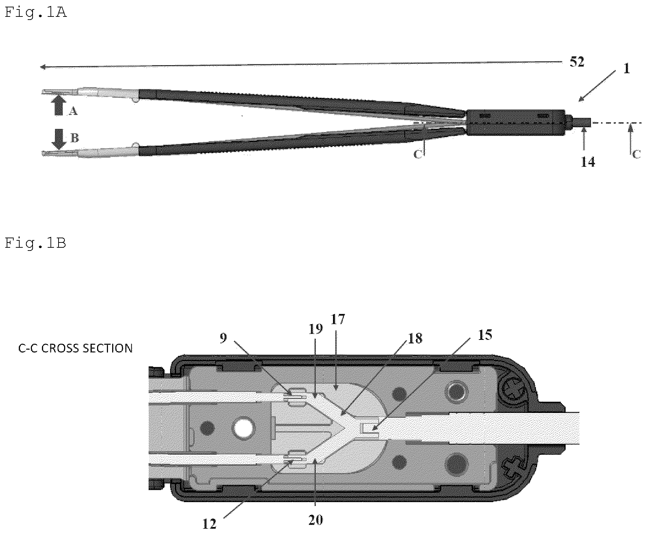

Hereinafter, the present invention is described with reference to the drawings. However, the present invention is not limited to a medical instrument illustrated in the drawings. Description is given while a view seen from a front side of FIG. 1A is regarded as a side view of a medical treatment instrument (1) of the present invention, and an upper side, a lower side, a left side, and a right side of each of the drawings as seen from the front side of each of the drawings are regarded as an upper side, a lower side, a left side, and a right side of the medical treatment instrument (1) of the present invention. In addition, an electrode side is regarded as a distal end side of the medical treatment instrument (1), and a microwave transmission coaxial cable (14) side is regarded as a base end side of the medical treatment instrument (1). A direction extending from the base end side to the distal end side (arrow (52)) is regarded as an extending direction of the medical treatment instrument (1).

With regard to the terms "fitting hole portion" and "fitting protruding portion" in the present invention, the shapes of a hole portion and a protruding portion are not particularly limited as long as the protruding portion can be fitted into the hole portion. For example, the protruding portion may be a protruding shape, a convex portion, or the like, and the hole portion may be a hole shape, a concave portion, a slit shape, or the like.

The term "engagement" in the present invention includes fitting, and it is not particularly limited as long as, for example, engagement portions couples to each other in such a manner as to be directly or indirectly in contact with each other.

### (Basic Configuration of Medical Treatment Instrument (1))

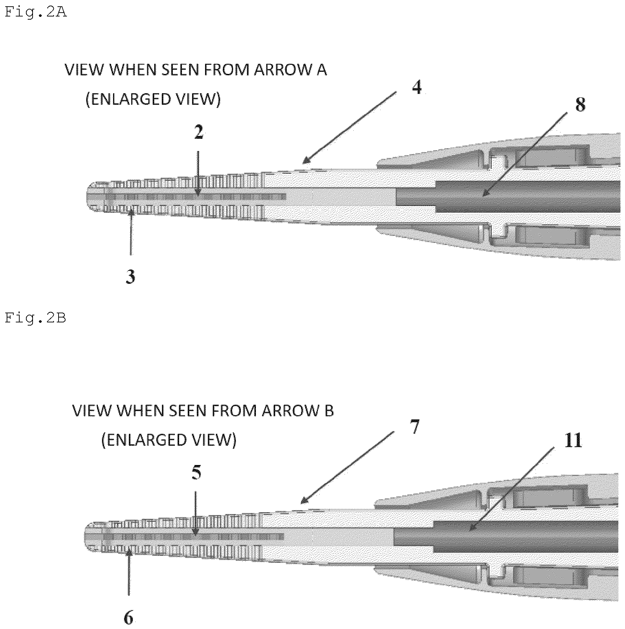

The basic configuration of the medical treatment instrument (1) of the present invention is not particularly limited as long as a microwave can be irradiated from both electrodes. The medical treatment instrument (1) has any one or more of the following configurations (see: FIGS. 1 to FIGS. 3).
- A first electrode (4) including a microwave application antenna A (2) and a microwave reception antenna B (3).
- A second electrode (7) including a microwave application antenna C (5) and a microwave reception antenna D (6).
- A first coaxial cable (8). The first coaxial cable (8) includes a first central conductor (9) and a first external conductor (10). A distal end of the first central conductor (9) and a distal end of the first external conductor (10) are directly or indirectly connected to a terminal end of the microwave application antenna A (2) and a terminal end of the microwave reception antenna B (3), respectively.
- A second coaxial cable (11). The second coaxial cable (11) includes a second central conductor (12) and a second external conductor (13). A distal end of the second central conductor (12) and a distal end of the second external conductor (13) are directly or indirectly connected to a terminal end of the microwave application antenna C (5) and a terminal end of the microwave reception antenna D (6), respectively.
- A microwave transmission coaxial cable (14). The microwave transmission coaxial cable (14) includes a central conductor (15) and an external conductor (16).
- A microwave splitter (17). The microwave splitter (17) includes a conductor for splitting (18) having a branch 1 (19) and a branch 2 (20). The branch 1 (19) and the branch 2 (20) have a two-legged fork shape on a distal end side of the microwave splitter (17). A terminal end of the conductor for splitting (18) is directly or indirectly connected to the central conductor (15) of the microwave transmission coaxial cable (14), a distal end of the branch 1 (19) is directly or indirectly connected to a terminal end of the first central conductor (9), and a distal end of the branch 2 (20) is directly or indirectly connected to a terminal end of the second central conductor (12) .

The medical treatment instrument (1) according to the present invention is not particularly limited as long as the medical treatment instrument (1) is movable such that tissue can be gripped in a gap defined between both electrodes which are the first electrode (4) and the second electrode (7), and examples thereof may include forceps and tweezers. As an example of the forceps, any publicly known forceps can be used as the forceps used in the present invention. Examples of the forceps used in the present invention may include Kelly forceps, Kocher forceps, Pean forceps, and Allis forceps. However, the forceps are not particularly limited.

### (Irradiation Microwave)

A microwave to be transmitted to the microwave transmission coaxial cable of the medical treatment instrument (1) according to the present invention is not particularly limited, but is from 300 MHz to 300 GHz (wavelength: from 1 m to 1 mm), preferably from 0. 9 GHz to 30 GHz. A transmission method can easily be achieved by any publicly known method, for example, a method of connecting the microwave transmission coaxial cable (14) to a publicly known microwave oscillator configured to oscillate a microwave or a method of incorporating the oscillator into the medical treatment instrument (1) and connecting such an oscillator to the microwave transmission coaxial cable (14).

Power used in the present invention is from 0.1 W to 200 W, preferably from 1.0 W to 80 W.

### (Coaxial Cable)

The coaxial cable used in the present invention is formed of, for example, a central conductor, a shield tube, and an earth pipe or a braided copper wire. The central conductor is an electric conductor made of copper. The shield tube is an insulator or a dielectric (which is made of, for example, Teflon (trademark) or polyethylene) configured to cover the central conductor. The earth pipe or the braided copper wire is an outer conductor (electric conductor) made of, for example, copper, stainless steel, or brass.

The coaxial cable may be a publicly known semi-rigid coaxial cable.

### (Antenna)

The microwave application antenna of the present invention is not particularly limited as long as the microwave application antenna is made of a material that enables supply of a microwave. A conductive material, such as silver, copper, gold, iron, titanium, stainless steel, phosphor bronze, or brass, may be widely used. Suitable examples thereof include silver, copper, gold, stainless steel, and brass.

The microwave reception antenna of the present invention is not particularly limited as long as the microwave reception antenna is made of a material that enables reception of a microwave. A conductive material, such as silver, copper, gold, iron, titanium, stainless steel, phosphor bronze, or brass, may be widely used. Suitable examples thereof include silver, copper, gold, stainless steel, and brass.

A shape of the antenna is not particularly limited. Examples of the shape of the antenna include a circular cone, a triangular pyramid, a quadrangular pyramid, a column, a quadrangular prism, a triangular prism, a sphere, a cube, and a cuboid. Further, for an inner surface (in particular, contact surface with respect to tissue) of the antenna, shapes such as, a blade shape, a planar shape, a cylindrical shape, a rod shape, a protrusion/recess shape, and a sawtooth shape, are widely applicable.

As a specific configuration of the microwave application antenna of the present invention, the quadrangular prism may be adopted, and the contact surface with respect to tissue may have the sawtooth shape so that tissue is less liable to slip. As a specific configuration of the microwave reception antenna, the quadrangular prism may be adopted, and the contact surface with respect to tissue may have the sawtooth shape so that tissue is less liable to slip.

### (Microwave Application Antenna)

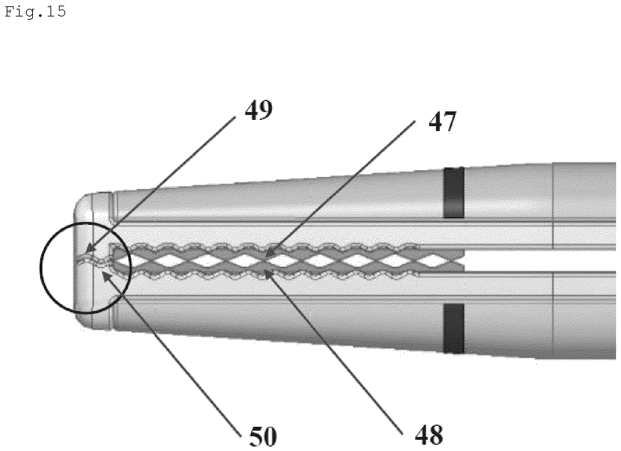

A surface of the microwave application antenna A (2) (surface in contact with the microwave application antenna C (5)) preferably has a sawtooth shape (47), and a surface of the microwave application antenna C (5) (surface in contact with the microwave application antenna A (2)) preferably has a sawtooth shape (48) (see: FIG. 15).

When the surface of the microwave application antenna A (2) and the surface of the microwave application antenna C (5) are brought into contact with each other, the surfaces contact with each other only with a blade of the sawtooth shape of the microwave application antenna A and a blade of the sawtooth shape of the microwave application antenna C.

With the configuration in which the surfaces of the antennas contact with each other only with the blades of the sawtooth shapes, compared with the case in which surfaces of the antennas contact with each other with the surfaces thereof being flat, a gripping force can be strengthened.

### (Microwave Reception Antenna)

A distal end surface of the microwave reception antenna B (3) (surface in contact with the microwave reception antenna D (6)) preferably has a tooth shape (49), and a distal end surface of the microwave reception antenna D (6) (surface in contact with the microwave reception antenna B (3)) preferably has a tooth shape (50).

Further, when the surface of the microwave reception antenna B (3) and the surface of the microwave reception antenna D (6) are brought into contact with each other, the surfaces mesh with each other only with the tooth shape (49) and the tooth shape (50).

With the distal ends of the antennas meshing with each other only with the tooth shapes of the respective distal ends (see: circle in FIG. 15), a thin tissue such as a membrane is easily grasped.

### (Electrode)

Further, the first electrode (4) and the second electrode (7) of the present invention are not particularly limited as long as the first electrode (4) and the second electrode (7) are movable so as to be capable of gripping tissue in the gap defined between both electrodes (in particular, forceps) and further have a coagulating function and a cutting function as needed.

It is more preferred that the electrodes each be partially or entirely subjected to coating that causes coagulated tissue to be less liable to adhere to the electrodes during contact with the tissue. The coating is performed with, for example, gold or a Teflon-based member. With this, processing of coagulation and cutting can be successively performed without causing adhesion of coagulated tissue.

A preferred configuration, more specifically, a configuration enabling efficient radiation of a microwave, of the first electrode (4) and/or the second electrode (7) of the present invention is described below.

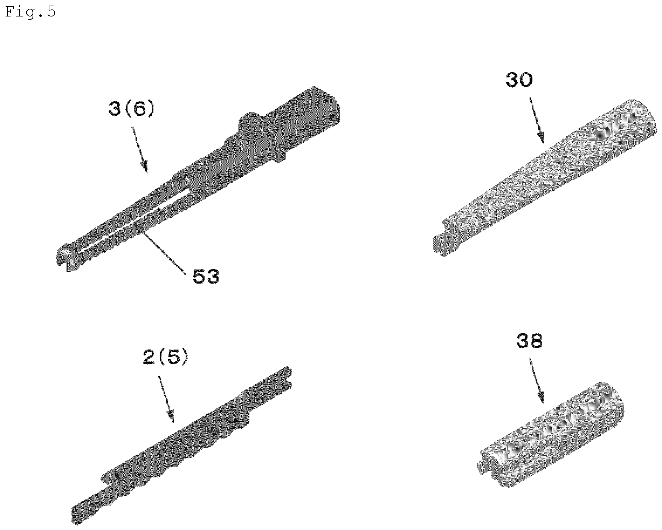

In the first electrode (4), the microwave application antenna A (2) is arranged in an internal space (53) of the microwave reception antenna B (3) through intermediation of an antenna cover A (30) (see: FIG. 5).

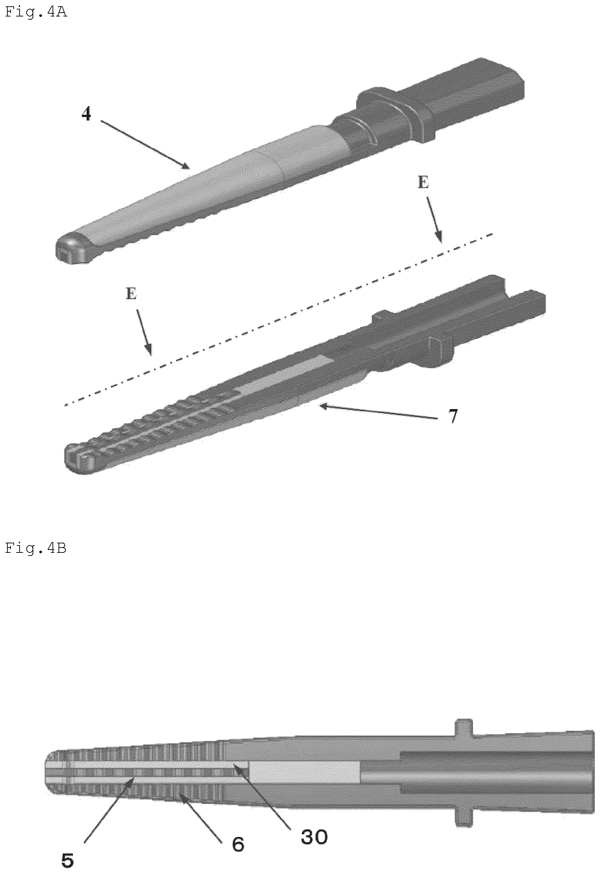

In the second electrode (7), the microwave application antenna C (5) is arranged in the internal space (53) of the microwave reception antenna D (6) through intermediation of the antenna cover A (30) (see: FIGS. 4 and FIG. 5).

### (Position Setting Portions (21) of First Electrode and Second Electrode)

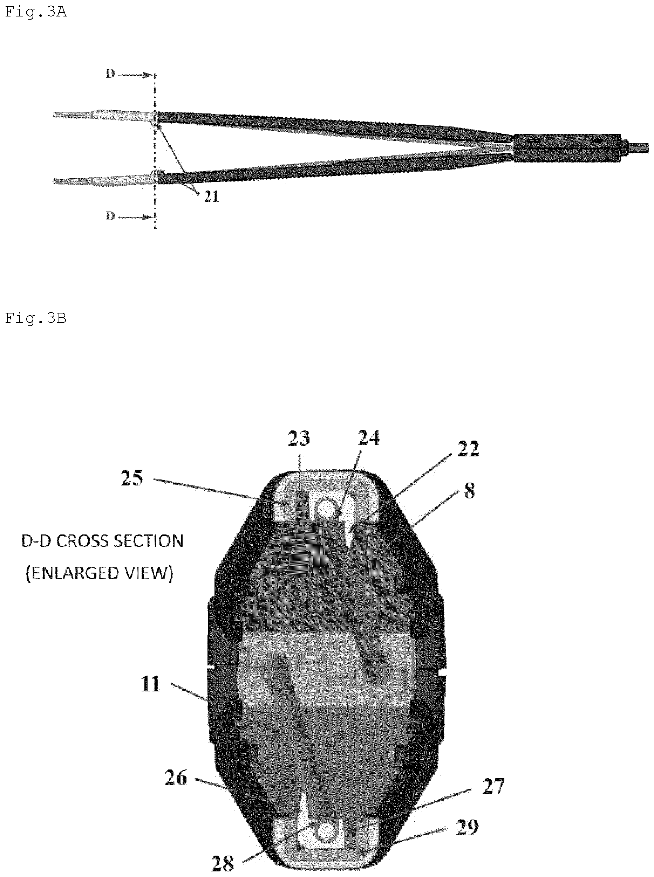

The medical treatment instrument (1) of the present invention preferably includes position setting portions (21) of a first electrode and a second electrode (see: FIGS. 3). The position setting portions (21) of the first electrode and the second electrode have a suppressive effect on occurrence of scissoring (occurrence of sparks).

The position setting portions (21) of the first electrode and the second electrode include a position setting portion A (25) and a position setting portion B (29) being opposed to each other. The position setting portion A (25) is arranged in the first electrode (4) including a fitting protruding portion A (22), a fitting hole portion A (23), and an accommodation portion A (24) that accommodates the first coaxial cable (8). The position setting portion B (29) is arranged in the second electrode including a fitting protruding portion B (26), a fitting hole portion B (27), and an accommodation portion B (28) that accommodates the second coaxial cable (11).

The position setting portions (21) of the first electrode and the second electrode are formed such that, when the first electrode (4) and the second electrode (7) are brought into contact with each other, the fitting protruding portion A (22) is accommodated in the fitting hole portion B (27), and the fitting protruding portion B (26) is accommodated in the fitting hole portion A (23). Specifically, the fitting protruding portion A (22) extends in a direction (downward direction) of being brought into contact with the second electrode, and the fitting protruding portion B (26) extends in a direction (upward direction) of being brought into contact with the first electrode.

The protruding portions are inserted into the respective hole portions. Accordingly, misalignment in a right-and-left direction can be prevented when seen from a front side of FIG. 3B in the case in which the first electrode (4) and the second electrode (7) are brought into contact with each other, thereby suppressing occurrence of scissoring (occurrence of sparks).

Further, with regard to an arrangement location of the position setting portion A (25), for example, the position setting portion A (25) is arranged at any position in a range between a terminal end of the microwave application antenna A (2) on a side in contact with the second electrode (7) of the first electrode (4) and a terminal end of the first electrode (4). More specifically, the arrangement location is preferably in a range of from the terminal end of the microwave application antenna A (2) to a terminal end of the first coaxial cable (8), more preferably in a range of from the terminal end of the microwave application antenna A (2) to a distal end of the first coaxial cable (8), in other words, the position setting portion A (25) is present in a range of from a distal end of the microwave application antenna A (2) to a position distant therefrom by from 10 mm to 60 mm (or 40 mm) (to the base end side).

Similarly, with respect to an arrangement location of the position setting portion B (29), for example, the position setting portion B (29) is arranged at any position in a range between the terminal end of the microwave application antenna C (5) on a side in contact with the first electrode (4) of the second electrode (7) and a terminal end of the second electrode (7). More specifically, the arrangement location is preferably in a range of from the terminal end of the microwave application antenna C (5) to a terminal end of the second coaxial cable (11), more preferably in a range of from the terminal end of the microwave application antenna C (5) to a distal end of the second coaxial cable (11), in other words, the position setting portion B (29) is present in a range of from a distal end of the microwave application antenna C (5) to a position distant therefrom by from 10 mm to 60 mm (or 40 mm) (to the base end side).

### (Antenna Cover A)

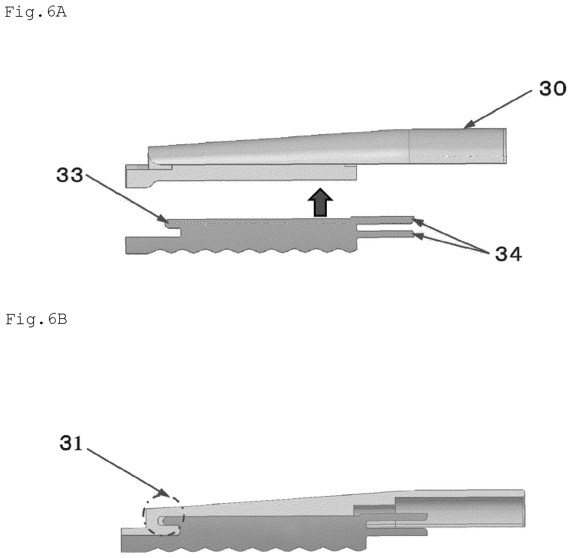

The medical treatment instrument (1) of the present invention preferably includes the antenna cover A (30). The antenna cover A (30) can prevent heat generation and radiation to a back side of a radiation surface (for example, the arrow direction of FIG. 6), which is in contact with the both electrodes.

The antenna cover A (30) includes a distal-end side inner cavity fitting hole portion (31) in an inner cavity on a distal end side of the cover.

Further, the microwave application antenna A (2) and/or the microwave application antenna C (5) includes a distal-end side fitting protruding portion (33) at an upper end on a distal end side of the antenna.

As illustrated in the dotted-line circle in FIG. 6B, the distal-end side fitting protruding portion (33) is fitted into the distal-end side inner cavity fitting hole portion (31), thereby being capable of preventing falling off of the microwave application antenna A (C) on the distal end side.

The antenna cover A (30) includes a distal-end side outer engagement portion (35) on the distal end side of the cover.

Further, the microwave reception antenna B (3) and/or the microwave reception antenna D (6) includes a receiving portion (36) on a distal end side of the antenna.

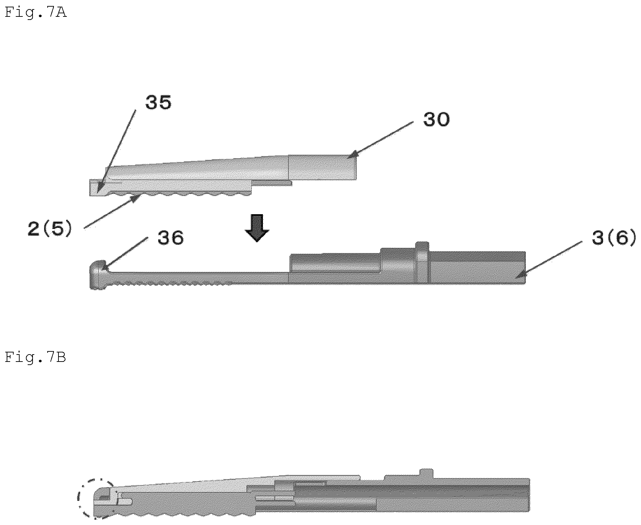

As illustrated in the dotted-line circle in FIG. 7B, the distal-end side outer engagement portion (35) is engaged with the distal-end side receiving portion (36), thereby being capable of preventing falling off of the antenna cover A (30) on a distal end side.

Although the material of the antenna cover A (30) is not particularly limited as long as heat generation and radiation caused by a microwave radiation can be prevented, an insulator or a dielectric can be exemplified. Further, polyether ether ketone (PEEK) can be exemplified.

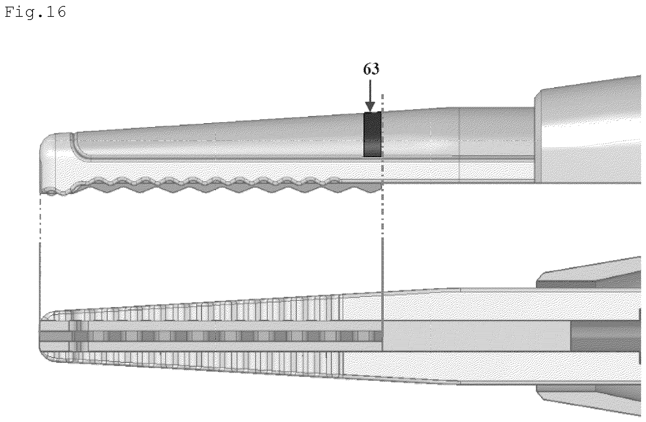

The antenna cover A preferably includes a marking (63) at a specific position from the distal end of the first electrode or the second electrode (in particular, the position of the terminal end of the microwave reception antenna) (see: FIG. 16) on a surface (outer surface) of the cover.

A user of the medical treatment instrument (1) of the present invention can easily grasp a microwave radiation range because of the position of the marking (63).

### (Antenna Cover B)

The medical treatment instrument (1) of the present invention preferably includes the antenna cover B (38).

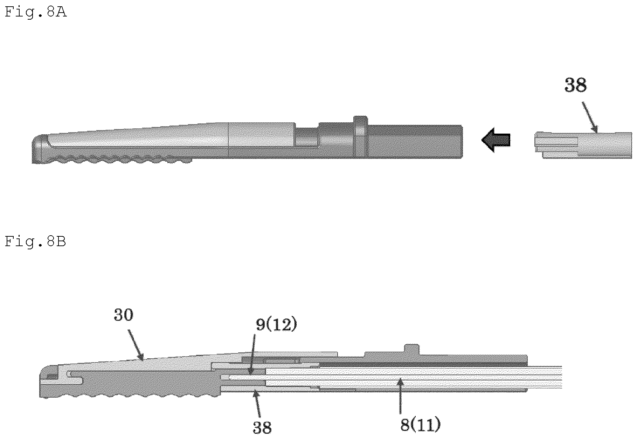

The antenna cover B (38) is located in an inner cavity of the microwave reception antenna B (3) and/or the microwave reception antenna D (6) (see: FIGS. 8).

The antenna cover B (38) preferably accommodates a part of the microwave application antenna A (2) and/or the microwave application antenna C (5) (two base-end side protruding portions (34)) on a terminal end side with the inner cavity of the cover (see: FIGS. 8).

As illustrated in FIG. 8B, the antenna cover B (38) accommodates the two base-end side protruding portions (34), thereby being capable of preventing falling off of the microwave application antenna A (C) on a base end side.

Although the material of the antenna cover B (38) is not particularly limited as long as heat generation and radiation caused by a microwave radiation can be prevented, an insulator or a dielectric can be exemplified. Further, polyether ether ketone (PEEK) can be exemplified.

The antenna cover B (38) preferably is formed into a key shape (40) having a round shape.

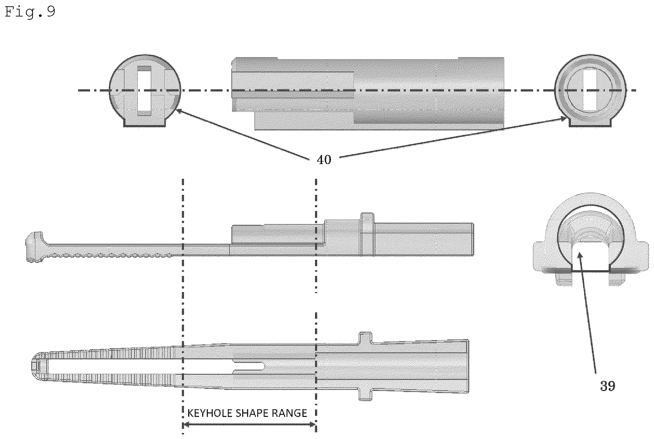

Further, in the microwave reception antenna B (3) and/or the microwave reception antenna D (6), the inner cavity extending from a base-end side opening portion of the antenna is preferably formed into a keyhole shape (39) corresponding to the key shape (40) having a round shape (see: FIG. 9).

The antenna cover B (38) is inserted into the microwave reception antenna B (3) and/or the microwave reception antenna D (6) from the base-end side opening portion so as to be located in the inner cavity of the antenna.

With regard to the key shape and the keyhole shape, as long as the antenna cover B (38) can be arranged in the inner cavity of the microwave reception antenna B (3) and/or the microwave reception antenna D (6), any publicly known shape can be adopted.

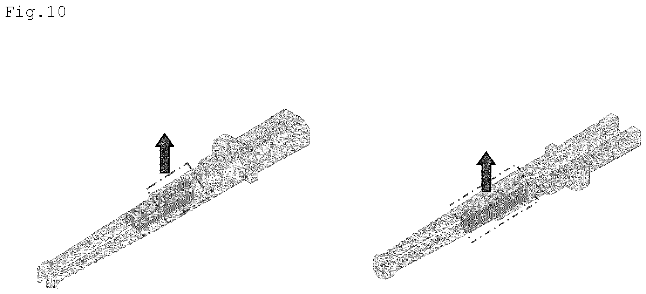

As illustrated on the left side of FIG. 10, with the round shape of the inner cavity of the microwave reception antenna B (3) and/or the microwave reception antenna D (6) and a round shape of an outer periphery of the antenna cover B (38), the antenna cover B (38) is prevented from moving in the arrow direction.

As illustrated on the left side of FIG. 10, with the key shape and the keyhole shape, the antenna cover B (38) is prevented from moving in the arrow direction.

More specifically, with the key shape and the keyhole shape, the antenna cover B (38) can move only in a right-and-left direction of FIGS. 8.

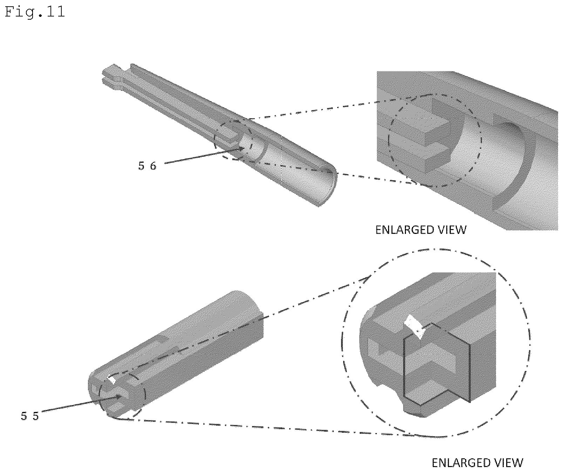

A distal end of the antenna cover B (38) preferably includes an engagement portion 1 (55) (see: FIG. 11).

The inner cavity of the antenna cover A (30) preferably includes an engagement portion 2 (56) (see: FIG. 11).

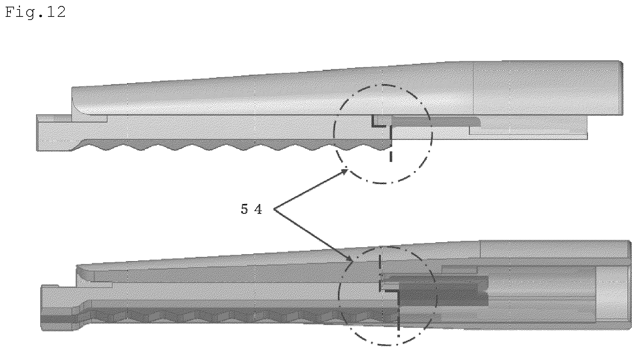

As illustrated in FIG. 12, the engagement portion 1 (55) and the engagement portion 2 (56) are engaged with each other. Specifically, the engagement portion 1 (55) has a protruding shape on an upper side of a distal end side (FIG. 12) and has a recessed shape on a lower side thereof (FIG. 12). The engagement portion 2 (55) has a recessed shape corresponding to the protruding shape of the engagement portion 1 (55) and has a protruding shape corresponding to the recessed shape of the engagement portion 1 (55). Thus, the antenna cover B (38) is prevented from moving in an upward direction or a downward direction of FIG. 12.

### (Microwave Splitter)

When the microwave splitter (17) of the present invention can split a microwave transmitted from the microwave transmission coaxial cable (14) to the first central conductor (9) and the second central conductor (12), any publicly known microwave splitter can be adopted. For example, a Wilkinson power divider, a 3-dB coupler splitter circuit, a rat-race splitter circuit, and a 90-degree hybrid splitter circuit can be exemplified.

Further, there may be arranged a device (circuit) required for matching an impedance of the microwave transmission coaxial cable (14) (microwave oscillation impedance) and an impedance of the first coaxial cable (8) and the second coaxial cable (11) with each other. Those circuits include a coil and a plurality of variable capacitors. Through adjustment of the electrostatic capacitance of the variable capacitors, or when those circuits include a stub, a sleeve, or the like that are variable in length, through adjustment of the length or the like of those components, impedance match can be performed.

### (Example of Microwave Splitter)

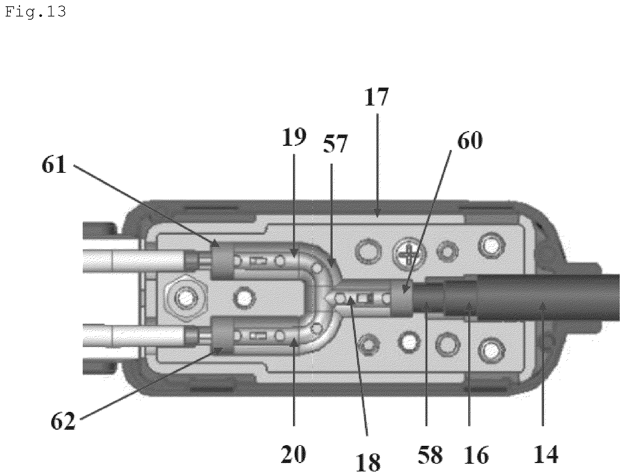

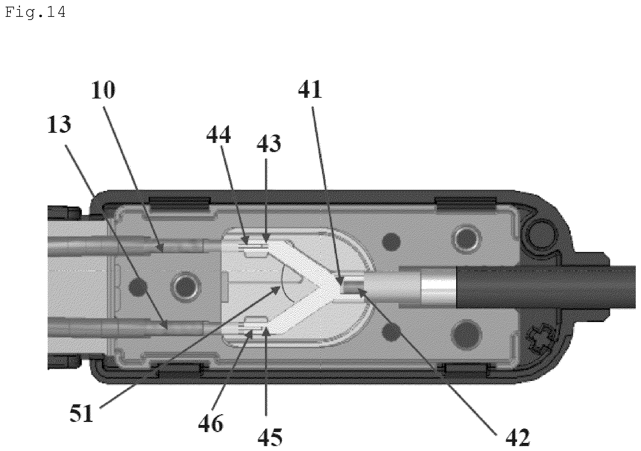

In the embodiment example of the microwave splitter (17) of the present invention (see: FIG. 13 and FIG. 14), the microwave splitter (17) is formed of the conductor for splitting (18). The conductor for splitting (18) having a two-legged fork shape is accommodated in a hollow tube (57) having a two-legged fork shape. Further, a casing (70) (case) in which those components are arranged (included) is preferably made of metal. Although the exterior of the casing is not particularly limited, a plastic material is more preferred.

A distal end of the central conductor (15) of the microwave transmission coaxial cable (14) including the central conductor (15), an insulator (dielectric) (58) that covers the central conductor, and the external conductor (16) that covers the insulator passes through a support column 1 (60) defining a space for the hollow tube (57) so that the distal end is directly or indirectly coupled to a terminal end (terminal end that is not branched into two legs) of the conductor for splitting (18).

A terminal end of the first central conductor (9) of the first coaxial cable (8) including the first central conductor (9), an insulator (dielectric) that covers the conductor, and the first external conductor (10) that covers the insulator passes through a support column 2 (61) defining the space for the hollow tube (57) so that the terminal end is directly or indirectly coupled to a distal end (one portion of a terminal end that is branched into two legs) of the conductor for splitting (18).

A terminal end of the second central conductor (12) of the second coaxial cable (11) including the second central conductor (12), an insulator (dielectric) that covers the conductor, and the second external conductor (13) that covers the insulator passes through a support column 3 (62) defining the space for the hollow tube (57) so that the terminal end is directly or indirectly coupled to a distal end (another portion of the terminal end that is branched into two legs) of the conductor for splitting (18).

Although the material of the central conductor is not particularly limited, copper, silver, gold, aluminum, and the like can be exemplified. Further, the fork shape may be a U-shape, a V-shape, or the like.

The external conductor is preferably a braided copper wire in order to prevent bending thereof.

The microwave splitter (17) of the present invention preferably includes a fitting hole portion (in particular, a slit) (41) in the terminal end of the conductor for splitting, and includes a fitting protruding portion (42) at the distal end of the central conductor (15).

The fitting protruding portion (42) at the distal end of the central conductor (15) is fitted into the fitting hole portion (41) in the terminal end of the conductor for splitting. Accordingly, separation of the coupling between the central conductor (15) and the conductor for splitting (18) can be suppressed.

The distal end of the branch 1 (19) of the conductor for splitting (18) includes a fitting hole portion (43) (in particular, a slit), and the terminal end of the first central conductor (9) includes a fitting protruding portion (44). The fitting protruding portion (44) is fitted into the fitting hole portion (43). Accordingly, separation of the coupling between the first central conductor (9) and the central conductor (15) can be suppressed.

The distal end of the branch 2 (20) of the conductor for splitting (18) includes a fitting hole portion (45) (in particular, a slit), and the terminal end of the second central conductor (12) includes a fitting protruding portion (46). The fitting protruding portion (46) is fitted into the fitting hole portion (45). Accordingly, separation of the coupling between the second central conductor (12) and the central conductor (15) can be suppressed.

The microwave splitter (17) of the present invention preferably has, unlike an internal angle (about 180°) of the related-art T-shaped conductor for splitting (18) illustrated in FIG. 13, an internal angle (51) formed by the branch 1 (19) and the branch 2 (20) having an angle of from 20° to 80°, more preferably, an angle of from 50° to 75°. With the range of the internal angle, a microwave can be efficiently irradiated to the branch 1 (19) and the branch 2 (20) .

As a manufacturing example of the conductor for splitting (18) having the internal angle described above, a copper metal plate having a gold-plated surface is preferably adopted.

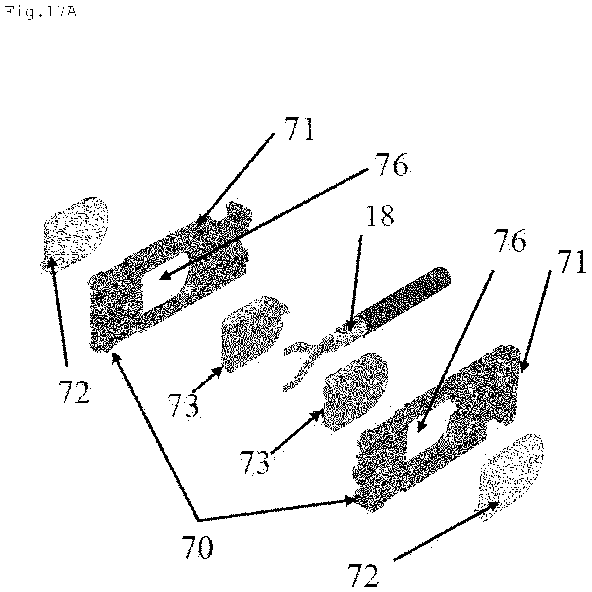

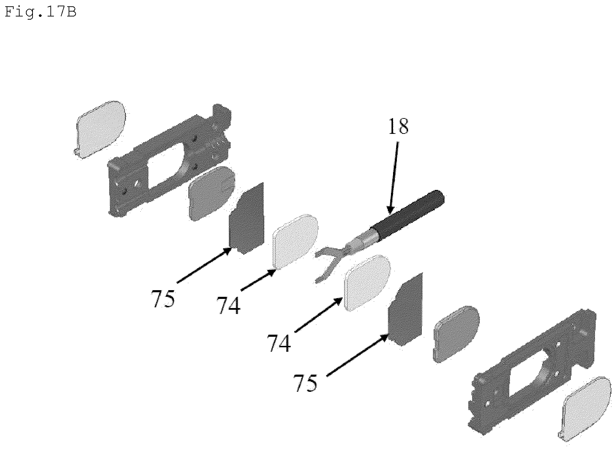

The configuration of the microwave splitter (17) of the present invention can be exemplified in FIG. 17A and FIG. 17B.

With regard to FIG. 17A, the casing (70) is formed so that two casing covers (71) sandwich therebetween the conductor for splitting (18) and insulator (dielectric) covers (73) from a substantially horizontal direction with respect to a flow direction of a microwave of the conductor for splitting (18). Further, the two casing covers (71) include cavities (76) defined in a substantially vertical direction with respect to the flow direction of a microwave, and outer covers (72) are arranged in the cavities (76).

Although the material of the outer covers (72) is not particularly limited as long as the outer covers (72) have an earth function in order to prevent leakage of a microwave, a stainless steel is preferred. The insulator (dielectric) cover (73) is arranged between the conductor for splitting (18) and the casing cover (71). As the material of the insulator (dielectric) cover (73), a polyether ether ketone resin (PEEK) can be exemplified.

With regard to FIG. 17B, in addition to the configuration of the microwave splitter (17) illustrated in FIG. 17A, the microwave splitter (17) includes two metal covers (75) and two resin covers (74).

The metal cover (75) is arranged between the resin cover (74) and the insulator (dielectric) cover (73). Although the material of the metal cover (75) is not particularly limited as long as the metal cover (75) has an earth function, a copper foil can be exemplified. The resin cover (74) is arranged between the conductor for splitting (18) and the metal cover (75). Although the material of the resin cover (74) is not particularly limited as long as the resin cover is made of a material that allows a dielectric constant in the casing (70) to be optimized, Teflon (trademark) (fluororesin) can be exemplified.

It has been confirmed that the microwave splitter (17) having the configuration illustrated in FIG. 17B has microwave radiation efficiency of about 1.5 times to about 2 times that of the microwave splitter (17) having a configuration illustrated in FIG. 17A.

### Industrial Applicability

The present invention can provide a medical treatment instrument in which occurrence of scissoring is suppressed.

### Reference Signs List

1. medical treatment instrument
2. microwave application antenna A
3. microwave reception antenna B
4. first electrode
5. microwave application antenna C
6. microwave reception antenna D
7. second electrode
8. first coaxial cable
9. first central conductor
10. first external conductor
11. second coaxial cable
12. second central conductor
13. second external conductor
14. microwave transmission coaxial cable
15. central conductor
16. external conductor
17. microwave splitter
18. conductor for splitting
19. branch 1
20. branch 2
21. position setting portion of first Electrode and second Electrode
22. fitting protruding portion A
23. fitting hole portion A
24. accommodation portion A
25. position setting portion A
26. fitting protruding portion B
27. fitting hole portion B
28. accommodation portion B
29. position setting portion B
30. antenna cover A
31. distal-end side inner cavity fitting hole portion of antenna cover A
33. distal-end side fitting protruding portion of microwave application antenna A and/or microwave application antenna C
34. two base-end side protruding portion of microwave application antenna A and/or microwave application antenna C
35. distal-end side outer engagement portion of antenna cover A
36. distal-end side receiving portion of microwave reception antenna B and/or microwave reception antenna D 38. antenna cover B
39. keyhole shape of microwave reception antenna B and/or microwave reception antenna D
40. key shape of antenna cover B
41. fitting hole portion in terminal end of conductor for splitting
42. fitting protruding portion in distal end of central conductor
43. fitting hole portion in distal end of branch 1
44. fitting protruding portion at terminal end of first central conductor
45. fitting hole portion in distal end of branch 2
46. fitting protruding portion at terminal end of second central conductor
47. sawtooth shape of microwave application antenna A
48. sawtooth shape of microwave application antenna C
49. tooth shape of microwave reception antenna B
50. tooth shape of microwave reception antenna D
51. internal angle formed by branch 1 and branch 2
52. arrow
53. internal space
54. engagement portion
55. engagement portion 1
56. engagement portion 2
57. hollow tube
58. insulator (dielectric)
60. support column 1
61. support column 2
62. support column 3
63. marking
70. casing
71. casing cover
72. outer cover
73. insulator (dielectric) cover
74. resin cover
75. metal cover
76. cavity

## Claims

1. A medical treatment instrument, comprising:
a first electrode including a microwave application antenna A and a microwave reception antenna B;
a second electrode including a microwave application antenna C and a microwave reception antenna D;
a first coaxial cable including a first central conductor and a first external conductor, a distal end of the first central conductor and a distal end of the first external conductor being directly or indirectly connected to a terminal end of the microwave application antenna A and a terminal end of the microwave reception antenna B, respectively;
a second coaxial cable including a second central conductor and a second external conductor, a distal end of the second central conductor and a distal end of the second external conductor being directly or indirectly connected to a terminal end of the microwave application antenna C and a terminal end of the microwave reception antenna D, respectively;
a microwave transmission coaxial cable including a central conductor and an external conductor;
a microwave splitter including a conductor for splitting having a branch 1 and a branch 2, the branch 1 and the branch 2 being formed into a two-legged fork shape on a distal end side of the microwave splitter, a terminal end of the conductor for splitting being directly or indirectly connected to the central conductor of the microwave transmission coaxial cable, the branch 1 being directly or indirectly connected to a terminal end of the first central conductor, and the branch 2 being directly or indirectly connected to a terminal end of the second central conductor; and
position setting portions of a first electrode and a second electrode, the position setting portions including a position setting portion A and a position setting portion B being opposed to each other, the position setting portion A being arranged in the first electrode including a fitting protruding portion A, a fitting hole portion A, and an accommodation portion A that accommodates the first coaxial cable, and a position setting portion B being arranged in the second electrode including a fitting protruding portion B, a fitting hole portion B, and an accommodation portion B that accommodates the second coaxial cable, the fitting protruding portion A and the fitting protruding portion B being configured to be accommodated in the fitting hole portion B and the fitting hole portion A, respectively, when the first electrode and the second electrode are brought into contact with each other.

2. The medical treatment instrument according to claim 1, further comprising an antenna cover A,
wherein the antenna cover A includes a distal-end side inner cavity fitting hole portion in an inner cavity on a distal end side of the cover,
wherein the microwave application antenna A and/or the microwave application antenna C includes a distal-end side fitting protruding portion at an upper end on a distal end side of the antenna, and
wherein the distal-end side fitting protruding portion is fitted into the distal-end side inner cavity fitting hole portion.

3. The medical treatment instrument according to claim 1 or 2,
wherein the antenna cover A includes a distal-end side outer engagement portion on a distal end side of the cover,
wherein the microwave reception antenna B and/or the microwave reception antenna D includes a distal-end side receiving portion of the antenna, and
wherein the distal-end side outer engagement portion is engaged with the distal-end side receiving portion.

4. The medical treatment instrument according to claim 2 or 3, further comprising an antenna cover B,
wherein the antenna cover B is located in an inner cavity of the microwave reception antenna B and/or the microwave reception antenna D, and accommodates a terminal end of the microwave application antenna A and/or the microwave application antenna C in an inner cavity of the cover.

5. The medical treatment instrument according to claim 4,
wherein the antenna cover B has a key shape, and
wherein the inner cavity of the microwave reception antenna B and/or the microwave reception antenna D has a keyhole shape corresponding to the key shape.

6. The medical treatment instrument according to claim 4 or 5,
wherein a distal end of the antenna cover B includes an engagement portion 1,
wherein an inner cavity of the antenna cover A includes an engagement portion 2, and
wherein the engagement portion 1 and the engagement portion 2 are engaged with each other.

7. The medical treatment instrument according to any one of claims 1 to 6,
wherein the terminal end of the conductor for splitting includes a fitting hole portion,
wherein a distal end of the central conductor includes a fitting protruding portion, and
wherein the fitting protruding portion is fitted into the fitting hole portion.

8. The medical treatment instrument according to any one of claims 1 to 7,
wherein a distal end of the branch 1 of the conductor for splitting includes a fitting hole portion, the terminal end of the first central conductor includes a fitting protruding portion, and the fitting protruding portion is fitted into the fitting hole portion, and
wherein a distal end of the branch 2 of conductor for splitting includes a fitting hole portion, the terminal end of the second central conductor includes a fitting protruding portion, and the fitting protruding portion is fitted into the fitting hole portion.

9. The medical treatment instrument according to any one of claims 1 to 8, wherein an internal angle formed by the branch 1 and the branch 2 is from 20° to 80°.

10. The medical treatment instrument according to any one of claims 1 to 9, wherein a surface of the microwave application antenna A and a surface of the microwave application antenna C each have a sawtooth shape, and are configured such that, when the first electrode and the second electrode are brought into contact with each other, the surfaces contact with each other only with a blade of the sawtooth shape of the microwave application antenna A and a blade of the sawtooth shape of the microwave application antenna C.

11. The medical treatment instrument according to any one of claims 1 to 10, wherein a distal end surface of the microwave reception antenna B and a distal end surface of the microwave reception antenna D each have a tooth shape, and are configured such that, when the first electrode and the second electrode are brought into contact with each other, the distal end surfaces mesh with each other only with the tooth shape of the distal end of the microwave reception antenna B and the tooth shape of the distal end of the microwave reception antenna D.

12. The medical treatment instrument according to any one of claims 1 to 11, wherein the antenna cover A includes a marking at a specific position from a distal end of the first electrode or the second electrode, on a surface of the cover A.

13. The medical treatment instrument according to any one of claims 1 to 12,
wherein the microwave splitter further includes:
a casing including two casing covers, the casing covers each having an outer cover arranged therein;
two insulator (dielectric) covers;
two metal covers; and
two resin covers,
wherein the two resin covers are arranged so as to sandwich the conductor for splitting therebetween,
wherein the two metal covers are arranged so as to sandwich the two resin covers therebetween,
wherein the two insulator (dielectric) covers are arranged so as to sandwich the two metal covers therebetween, and
wherein the two casing covers are configured so as to sandwich the two insulator (dielectric) covers therebetween.
